# EUROPEAN PATENT APPLICATION

(11) **EP 1 982 659 A2**
(43) Date of publication of application: **22.10.2008**
(21) Application number: 07254850.6
(22) Date of filing: 13.12.2007
(51) Int. Cl.: A61B 17/132

(54) **Method and apparatus for restricting blood flow**

(30) Priority: 20.04.2007 US 788469
(71) Applicant: O'Neil, Terence, Newport Coast CA 92657 (US)
(72) Inventor: O'Neil, Terence, Newport Coast CA 92657 (US)
(74) Representative: McLeish, Nicholas Alistair Maxwell

(57) **Abstract**

The present invention is an apparatus and method for applying a tourniquet device to an injured appendage requiring only one hand comprising the steps of providing a releasable cable tie (10) having an elongate band portion (12) with regularly spaced grooves (14) or projections on a first surface and a relatively smooth second surface, and a shroud member (18) disposed at a proximal end of said elongate band portion adapted to receive the distal end of the band portion and having a releasable groove or projection engaging element incorporated (24) therein, forming a loop with said elongate band member such that the relatively smooth surface is facing radially inward and the regularly spaced grooves (14) open radially outward, maneuvering the loop about the injured appendage, inserting the distal end of the elongate band (12) into the shroud member (18) such that the groove or projection engaging member is adjacent the grooves or projections (14), pulling the distal end of the elongate band (12) through the shroud member (18) until the requisite restriction of blood flow is achieved, and then releasing the groove engaging member after attending to the wound.

## Description

### BACKGROUND OF THE INVENTION

This invention relates in general to the field of blood flow restriction devices such as tourniquets, and more particularly to an apparatus and method of restricting blood flow in an appendage such as an arm or finger quickly and reliably using a simple device that constricts and releases the appendage as required.

Tourniquets are well known for the purpose of temporarily restricting the flow of blood to a person's injured limb in order to prevent a serious loss of blood. In battle environments, many deaths are attributed to blood loss from extremities as a result of body armor that is applied around the trunk. Death can occur within minutes due to blood loss, so the time needed to apply a tourniquet is critical. Further, it is most advantageous if a tourniquet can be applied by the victim, but self application of a tourniquet has been at best problematic in the past. Most emergency medical technicians and paramedics have belts or cords that can be fashioned into a tourniquet when needed. However, these devices suffer from one drawback or another in that they either require the use of both hands to secure and tighten, or they are difficult to thread about the injured appendage. Further, where the injury occurs in the home or workplace and unskilled persons or the injured party themselves may be required to apply the tourniquet, previous devices and methods do not lend themselves to easy application and secure blood flow restriction.

### SUMMARY OF THE INVENTION

The device used in the present invention is light-weight, sterile, easy portable, strong, reliable, and cost effective. The method of the present invention using this device is well suited for the battlefield, emergency situations in isolated areas, as well as more common emergency environments. It can also be easily and quickly applied by the victim in a reliable manner. An important feature of the present invention is that the wounded appendage does not have to be moved to apply the device, since it can be slid under the appendage and secured without the need to either lift the appendance or otherwise move it. This feature can be critical if the victim is trapped in a situation where either the victim is immobile or cannot be easily accessed, as is the case in automobile accidents and accidents involving machinery. The present method can also be used in medical procedures and surgeries where a tourniquet is involved and can replace more expensive apparatus that performs the same function.

The present invention is a method for applying a tourniquet device to an injured appendage requiring only one hand comprising the steps of providing a releasable cable tie having an elongate band portion having regularly spaced grooves on a first surface and a relatively smooth second surface, and a shroud member disposed at a proximal end of said elongate band portion adapted to receive the distal end of the band portion and having a releasable groove engaging element incorporated therein, forming a loop with said elongate band member such that the relatively smooth surface is facing radially inward and the regularly spaced grooves open radially outward, maneuvering the loop about the injured appendage, inserting the distal end of the elongate band into the shroud member such that the groove engaging member is adjacent the grooves, pulling the distal end of the elongate band through the shroud member until the requisite restriction of blood flow is achieved, and then releasing the groove engaging member after attending to the wound.

The method can also be adapted to apply direct pressure to a wound when direct pressure cannot otherwise be applied to the area. This is done by placing gauze on the wound and then gently applying direct pressure with the device as described above.

Blood flow constricting devices should not ordinarily be left in place for over one hour, as they may cause neurovascular damage. The unique device of the present invention allows the tourniquet to be released for a time period, and then retightened, mitigating prolonged ischemia time. Since the device is releasable, the pressure can be titrated and readjusted to provide only the minimum amount of pressure needed. The readjustable nature of the device is particularly useful for medical procedures, such as finger lacerations and ingrown toe nails, as these are particularly vascular areas. Current standard of care is to inject a local anesthetic without epinephrine, as epinephrine has been found to cause tissue necrosis in digits. The addition of epinephrine would normally decrease blood flow to an area decreasing the need for a tourniquet, as the area where one would be working could more easily be visualized. However, as epinephrine cannot be used with the local anesthetic, a tourniquet is often needed to decrease bleeding in order to visualize a particular area. A tourniquet needs to be applied to digits (fingers and toes) due to their highly vascular nature.

Presently a rubber band, Penrose drain, or the finger of a rubber glove is used with a clamp as a digital tourniquet. The hazard with these types of tourniquets is that they tend to roll up as they are stretched, transmitting force over a very small area. This concentrated force increases the risk of neurovascular damage. Furthermore, these tourniquets do not allow the amount of pressure being applied for controlling bleeding to be easily modified. As a result, they tend to be applied too tightly causing undue force on the digit, increasing the risk of neurovascular injury. The present invention allows for an easy, low cost way to apply the minimal amount of pressure in order to achieve vascular control. The amount of pressure can be increased or decreased easily and more accurately than previous methods using the releasable locking mechanism. In addition, the rigid nature of the apparatus prevents it from rolling up and narrowing the area of applied force, mitigating any potential neurovascular compromise.

The present invention is well suited for medical procedures where a tourniquet is required, such as surgeries on the hand, arm, foot, ankle, knee, or other extremities. Once again, the pressure can easily be titrated to obtain the minimal amount of pressure needed. Additionally, it facilitates procedures outside of an operating room as expensive equipment is not needed to obtain vascular control.

Other features and advantages of the invention will become apparent from the following detailed description, taken in conjunction with the accompanying drawings which illustrate, by way of example, the features of the invention.

### BRIEF DESCRIPTION OF THE DRAWINGS

Figure 1 is a plan view of a preferred blood flow constricting device used in the present invention;

Fig. 2 is a side view of the device of Fig. 1;

Fig. 3 depicts the blood flow constricting device of Fig. 1 being formed into a loop;

Fig. 4 depicts the loop illustrated in Fig. 3 being placed over a finger;

Fig. 5 depicts the device of Fig. 1 being tightened about the finger;

Fig. 6 depicts the release of the blood flow constricting device;

Fig. 7 depicts one locking arrangement for the device depicted in Fig. 1; and

Fig. 8 depicts the locking arrangement being released.

### DETAILED DESCRIPTION OF THE PREFERRED EMBODIMENTS

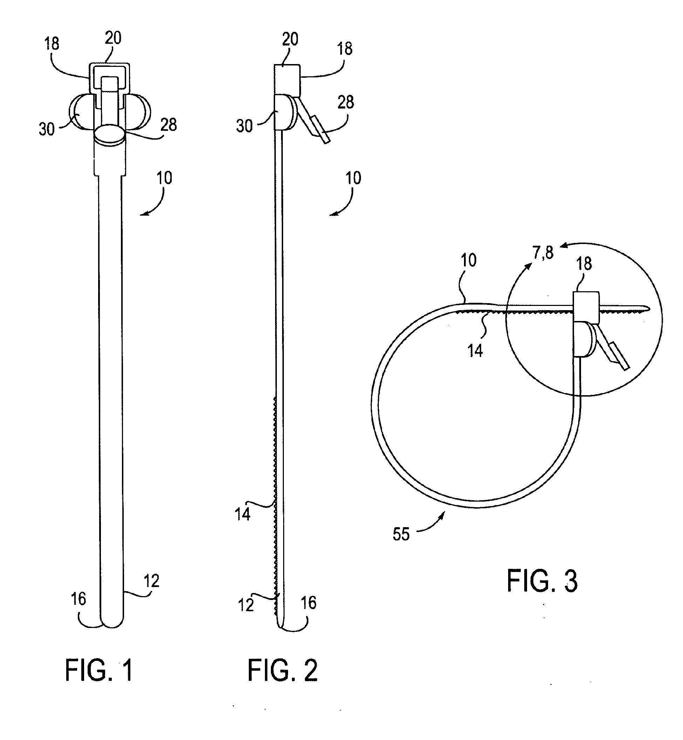

For a better understanding of the present invention together with other and further objects, advantages and capabilities thereof, reference is made to the following disclosure and appended claims in connection with the above described drawings. Figure 1 illustrates a releasable cable tie 10 of the present invention which operates in a manner such as certain cable ties offered for construction applications like those offered by KAI SUH SUH Enterprise Co., Ltd., as shown at http://www.allproducts.com/ee/kss/cable_tie.html. The cable tie 10 may be made from Nylon or plastic which has sufficient rigidity to prevent the tie from folding or rolling as pressure is applied, although any suitable material can achieve the objectives of the present invention. The cable tie 10 is preferably a single use application where the tie is sterile and enclosed in a sealed package until its ready for use. It is well suited for many locations, such as operating rooms, emergency rooms, urgent care centers, clinics and doctor's offices.

The cable tie 10 includes an elongate band 12 that includes a flat, smooth surface on one side and spaced apart grooves 14 on the opposite side extending laterally across the band 12 in regular intervals. The distal end 16 of the tie 10 my have a narrowing tip to facilitate insertion through the shroud 18 at the proximal end 20 of the tie 10. As the distal end 16 of the tie 10 is inserted the shroud 18, the grooves 14 are sequentially engaged by a lever arm 22 disposed over an opening in the shroud 18. The lever arm 22 includes a tab or projection 24 that inserts into each groove 14 as the tie 10 passes through the shroud 18 and prevents the tie from being withdrawn back through the shroud. This ensures that once the tie 10 is pulled to a particular loop size or pressure, the loop will not release as long as the lever arm 22 is not disengaged from the occupied groove. The lever arm 22 is resilient, but rigid enough to engage the grooves 14 and resist the tie being withdrawn unless released by a release button 28. Alternatively, the grooves 14 on the strap 12 can be replaced by projecting teeth and the lever arm 22 can be adapted with a slot or groove to receive one or more teeth to lock the lever arm in place and fix the tie. The proximal end 20 of the tie 10 may further include right and left wings 30 projecting laterally outward from the tie and may be inclined slightly upward to provide a platform from which the tie can be maneuvered to release the lever arm 22. The lever arm 22 is biased downward by the shape memory of the resilient lever arm 22 to automatically engage the grooves 14 passing below, and may be either pivoted away from the opening in the shroud 18 against the biasing by pressing down on the release button 28 to unlock the tie.

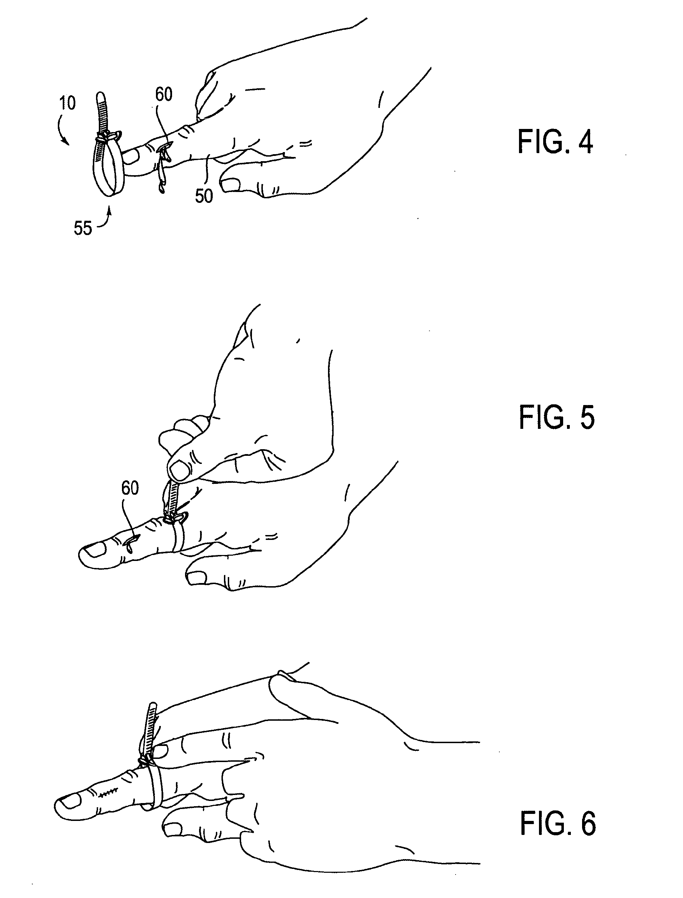

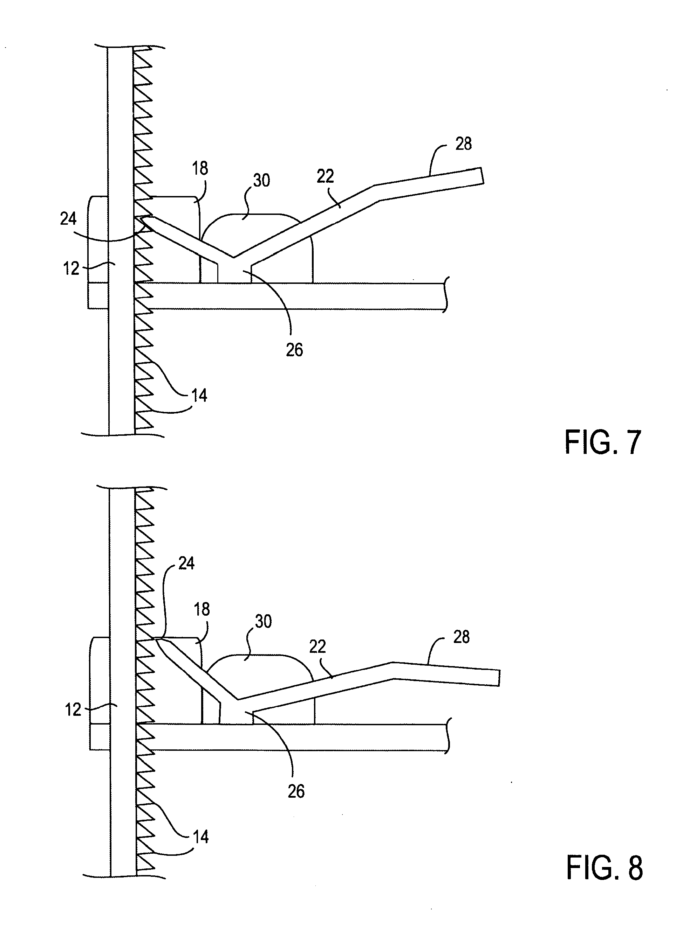

In operation as shown in Figs. 3-6, when the tie 10 is needed to restrict blood flow in an appendage such as a finger 50, the tie is preferably removed from a sterile packaging and brought in proximity with the patient. With the grooved surface facing upward, the distal end 16 of the tie 10 is bent backwards and inserted into the shroud to form a loop 55 (Fig. 3). The loop 55 is then placed over the appendage 50 between the wound/surgical location 60 and the victim's heart (not shown) so as to be in position to restrict blood flow to the area (Fig. 4). The distal end 16 of the tie 10 is pulled slowly through the shroud 18, where the flexible lever arm 22 sequentially engages the grooves 14 (see Fig. 7) to prevent release of the loop and accompanying pressure (Fig. 5). The tie 10 is tightened around the appendage 50 until the requisite amount of pressure is applied to restrict the flow of blood to the designated area, whereupon the pulling of the tie is ceased. The lever arm 22 engages the groove 14 immediately below and prevents the tie from receding back through the shroud 18 to ensure constant and secure pressure about the appendage 50. If the flow of blood requires adjusting, the flow can be restricted further by simple pulling the tie 10 one groove 14 at a time until the correct pressure is achieved. If the pressure needs to be lessened, the lever arm 22 can be released by actuating the release button 28 to disengage the lever arm (Fig. 8) from the grooves 14, to allow the tie to be withdrawn a notch or two to broaden the loop 55. When the need for restricting the blood flow has subsided, the releasable aspect of the invention allows the lever arm 22 to be disengaged and the distal end 16 of the tie 10 pulled through the shroud 18 until it is completely withdrawn.

The figures depict the various steps involved in the novel process. The tie is formed into a loop 55 in Fig. 3 by placing the distal end 16 of the tie 10 into the shroud 18 and pulling the end through the other side. The loop 55 is then placed over the appendage 50 to be restricted in Fig. 4, and the loop 55 is tightened where the lever arm 22 engages the grooves 14 at regular intervals to prevent expansion of the loop 55 and secure the tie. When the loop 55 is securely placed around the appendage 50 and the flow of blood sufficiently restricted, the lever arm 22 or other engagement prevents slippage until the tourniquet is ready to be released. In Fig. 6, the lever arm 22 is retracted by pressing down on the release button 28 to disengage the lever arm 22 from the grooves 14 or teeth, and the loop 55 is opened to allow blood flow to resume normally.

The method and apparatus just described is illustrative of the present invention, and should not be deemed to be limiting in any manner. Rather, the scope of the invention is properly considered to include all variations and modifications that would be considered by those of ordinary skill in the art. Accordingly, the invention is properly measured not by the aforementioned description but rather by the words of the appended claims and all equivalents attributable thereto.

## Claims

1. A method for constricting blood flow through an appendage comprising the steps of:
providing a blood flow constricting device comprising an elongate band having a flat smooth surface on a first side, and a second side having regularly spaced apart projections extending transversely across said elongate band, and a shroud member disposed at a proximal end of said band including a channel sized for receiving said band member therethrough and a releasable locking member for engaging a projection to releasably lock said band within said shroud member, said locking member including a manually actuated release that disengages the projection from the elongate band;
placing a distal end of said band into said shroud member to form a loop such that said flat smooth surface is directed radially inward and said spaced apart projections are directed radially outward;
placing the loop around said appendage;
pulling the distal end of said band to reduce the loop until a tension is achieved corresponding to a requisite restriction of blood flow, where passage of said elongate band through said shroud member causes said locking member to engage each projection passing thereby;
releasing the distal end of the band, whereupon the releasable locking member engages a most proximate projection to retain the requisite tension in the loop about the appendage; and
actuating the locking member's release to disengage the projection and unload the tension in said loop after providing necessary medical attention to the appendage.

2. The method of Claim 1 further comprising the step of initially removing the blood flow constricting device from a sterile packaging.

3. The method of Claim 1 further comprising the step of titrating the pressure after releasing the distal end of the band to adjust the requisite pressure as needed.

4. The method of Claim 1 wherein the step of actuating the locking member's release is accomplished by pressing an end of the locking member with a finger.

5. A method for constricting blood flow through an appendage comprising the steps of:
providing a blood flow constricting device comprising an elongate band having a flat smooth surface on a first side, and a second side having regularly spaced apart grooves extending transversely across said elongate band, and a shroud member disposed at a proximal end of said band including a channel sized for receiving said band member therethrough and a releasable locking member for engaging a groove to releasably lock said band within said shroud member, said locking member including a manually actuated release that disengages the locking member from the groove;
placing a distal end of said band into said shroud member to form a loop such that said flat smooth surface is directed radially inward and said spaced apart grooves are directed radially outward;
placing the loop around said appendage;
pulling the distal end of said band to reduce the loop until a tension is achieved corresponding to a requisite restriction of blood flow, where passage of said elongate band through said shroud member causes said locking member to engage each groove passing thereby;
releasing the distal end of the band, whereupon the releasable locking member engages a most proximate groove to retain the requisite tension in the loop about the appendage; and
actuating the locking member's release to disengage the locking member and unload the tension in said loop after providing necessary medical attention to the appendage.

6. The method of Claim 1 further comprising the step of initially removing the blood flow constricting device from a sterile packaging.

7. The method of Claim 1 further comprising the step of titrating the pressure after releasing the distal end of the band to adjust the requisite pressure as needed.

8. The method of Claim 1 wherein the step of actuating the locking member's release is accomplished by pressing an end of the locking member with a finger.

9. A releasable blood flow restriction device comprising:
an elongate band having a series of closely spaced projections extending laterally on at least one surface;
first and second wing members inclined from a base of said band;
a shroud at a first end of said base sized to receive said elongate band therein to form a loop of adjustable size for encircling an appendage;
projection engagement means disposed adjacent said shroud for engaging said projections to lock said loop at a particular size to restrict the flow of blood in the appendage; and
a release lever coupled to said projection engagement means to release the projection engagement means so as to reduce a pressure on said appendage by said loop, said release lever including an enlarged tab member to facilitate actuating said release lever.
